# NEW EUROPEAN PATENT SPECIFICATION

(11) **EP 1 828 011 B2**
(45) Date of publication and mention of the opposition decision: **31.07.2013**
(45) Mention of the grant of the patent: 21.10.2009
(21) Application number: 05852333.3
(22) Date of filing: 28.11.2005
(51) Int. Cl.: B65D 75/52, A61F 15/00

(54) **PACKAGE AND DISPENSING SYSTEM FOR PERSONAL CARE ARTICLES**
VERPACKUNG UND AUSGABESYSTEM FÜR KÖRPERPFLEGEARTIKEL
PAQUET ET SYSTEME DE DISTRIBUTION POUR DES ARTICLES DE SOINS PERSONNELS

(30) Priority: 23.12.2004 US 20724
(43) Date of publication of application: 05.09.2007
(73) Proprietor: KIMBERLY-CLARK WORLDWIDE, INC., Neenah, WI 54956 (US)
(72) Inventor: ZANDER, Teresa, Marie, Bonduel, Wisconsin 54107 (US); BRYANT, Kristi, Jo, Appleton, Wisconsin 54914 (US); WHEELER, Katherine, Carol, Menasha, Wisconsin 54952 (US); VELAZQUEZ, Herb, Flores, Neenah, Wisconsin 54956 (US)
(74) Representative: Davies, Christopher Robert
(86) International application number: PCT/US2005/043004
(87) International publication number: WO 2006/071424

(56) References cited:
- EP-A1- 0 360 925
- EP-A1- 1 588 682
- DE-U1- 8 705 107
- GB-A- 410 460
- GB-B- 2 395 929
- US-A- 2 815 126
- US-A- 3 353 661
- US-A- 4 564 108
- US-A1- 2002 060 167
- US-A1- 2003 004 089
- US-A1- 2003 047 935
- US-A1- 2003 102 239
- US-A1- 2003 120 241
- US-A1- 2004 136 774

## Description

### FIELD OF THE INVENTION

The present invention generally relates to a package and dispensing system for articles, in particular, absorbent personal care articles.

### BACKGROUND OF THE INVENTION

Absorbent personal care articles, such as pantiliners, feminine napkins, incontinence products, tampons and the like, are frequently carried about in purses, backpacks, briefcases, overnight bags and the like until needed. Generally, the absorbent personal care products are wrapped in a pouch or similar wrapper to help protect the absorbent personal care article before use. The pouch or similar wrapper helps maintain the sanitary nature of the absorbent personal care before use. Typically, each pouch or wrapper contains a single absorbent personal care article; however, there are some commercially available personal care products with a plurality of absorbent personal care articles in a single pouch or wrapper. Often, the absorbent personal care articles, wrapped in a pouch or wrapper are placed in purses, backpacks, briefcases, overnight bags, and the like, such that they are loose, and are free to move within the purses, backpacks, briefcases, overnight bags, and the like with the other items contained therein. Often this can result in damage to the pouch or wrapper and sometimes the absorbent personal care article. Further, the articles can become scattered about in purses, backpacks, briefcases, overnight bags and the like so they are difficult to find when needed.

In the past, specifically designed purse-like containers have been distributed for holding several absorbent articles at a time. These containers reduce contact between the articles and potentially non-hygienic environments, and make the articles easier to find when needed. The containers are typically made from durable materials such as heavy vinyl so they can be reused, but reuse necessitates the container is refilled from time to time. Generally, theses containers are envelope type containers with a closure device. Many times, the entire container must be removed from the purse, backpack, briefcase, overnight bag and the like, in order to access the absorbent personal care articles. Further, the pouches frequently become non-hygienic after extended use, requiring them to be cleaned or discarded and replaced. As a result, there is a need for a package for absorbent personal care articles, which does not have to be refilled, protects the absorbent personal care product and pouch or wrapper from damage when in purses, backpacks, briefcases, overnight bags and the like, and provides the user of the personal care products easy access to the personal care product when needed.

In addition, absorbent personal care articles are often provided in a flexible package, typically made of a film or some other type of flexible packing material. Packaging bags made from flexible polymeric materials have been used for packaging various types of products, including, for example, adult incontinence articles, diapers, training pants, feminine care articles, among many other items. These bags provide packaging for the products, creating a carton-like look and configuration which facilitates display of the products on the retail shelf for consumers to purchase. These bags also provide a convenient way for the consumer to transport the products from the retailer to the consumer's home or place of use. However, these packaging bags can be difficult to store especially for consumers who live in older homes, or who live in relatively cramped living quarters, such as college dorms, small apartments and the like. This is because closed storage in bathrooms or bedrooms which is large enough to store a package of personal care articles is many times not available or is available on a limited basis. As a result, many times the absorbent personal care products are removed from the package and placed in a drawer or other available storage, along with other personal care items. This can result in damage to the pouch or wrapper and sometimes the absorbent personal care article. Further, the absorbent personal care articles can become scattered about in a drawer or similar storage so they are difficult to find when needed. Therefore, there is a need in the art for an alternative way to purchase and store absorbent personal care articles for some users of these products.
A prior art package, EP-A-036092S described a package of tissues in booklet form, is shown in US-2 815 126.

### SUMMARY OF THE INVENTION

The present invention provides a package as set out in claim 1. The package has a plurality absorbent personal care articles and a plurality packaging components. Each of the packaging components has a compartment, a fixed end and a free end. Each fixed end is connected directly or indirectly to at least one other fixed end of another packaging component and each free end of a given packaging component is opposed to the fixed end. The compartment of each packaging component has at least one absorbent personal care article placed within the compartment. The package forms a look-like structure. The packaging component are removable from the package.

The package of the present invention may optionally have a cover to further protect the packaging components and absorbent articles contained therein. Other features provided in the package of the present invention include a fastening means which allows the package to remain in a closed position. The fastening means also provides a way to hang the package on a door or a wall, as well as to secure the package within a cabinet drawer.

### BRIEF DESCRIPTION OF THE DRAWINGS

FIGURE 1 is a body-side plan view of an exemplary personal care product component with a portion thereof partially cut away.

FIGURE 2 is a perspective view of a product component in a partially folded configuration.

FIGURE 3 shows a perspective view of a package of the present invention

FIGURE 4 shows a top view of a package of the present invention.

FIGURE 5A shows a plan view of a packaging component in a open position which is usable in the package of the present invention.

FIGURE 5B shows a plan view of a packaging component in an closed position which is usable in the package of the present invention.

FIGURE 6A shows a plan view of a packaging component in a closed position which is usable in the package of the present invention.

FIGURE 6B shows a plan view of a packaging component in a closed position which is usable in the package of the present invention.

FIGURE 6C shows a plan view of a packaging component outside the scope of the claims which dispenses the article when opened or removed from the package.

FIGURE 6D shows a plan view of a packaging component outside the scope of the claims of FIGURE 6C dispensing an article from the package as the packaging component is opened.

FIGURE 7A shows a plan view of a packaging component with more than one compartment.

FIGURE 7B shows a plan view of another packaging component with more than one compartment.

FIGURE 8A shows a spine configuration for indirectly attaching the fixed end of the individual packaging components together.

FIGURE 8B shows a spine configuration outside the scope of the claims for directly attaching the fixed end of the individual packaging components together.

### DEFINITIONS

It should be noted that, when employed in the present disclosure, the terms "comprises", "comprising" and other derivatives from the root term "comprise" are intended to be open-ended terms that specify the presence of any stated features, elements, integers, steps, or components, and are not intended to preclude the presence or addition of one or more other features, elements, integers, steps, components, or groups thereof.

It should be understood that the term "personal care product" or "personal care article" as used herein refers to any article used to control bodily fluids, and includes "absorbent articles," which refers to any article configured to absorb and retain bodily exudates, including urine, bowel movements, blood and menses, and includes such a product in a packaged and unpackaged configuration. As such, personal care products, as used herein, includes without limitation, diapers, child toilet training pants, adult incontinence garments, male incontinence products, tampons, vaginal suppositories, pantiliners, pads, sanitary napkins, tissues, wipes, etc. Examples of commercially available personal care products include, without limitation, Poise® feminine care products, including pantiliners and pads, and Kotex® feminine care products, including pads, tampons and liners, Depend ® undergarments, underwear and guards, all available from Kimberly-Clark Corporation, Neenah, Wisconsin.

As used herein, the term "fixed end" is intended to mean an end of the packaging component or cover, if present, which is attached, directly or indirectly, to the package.

As used herein, the term "free end" is intended to mean an end of the packaging component or cover, if present, which is opposite or opposed to the fixed end. Generally the free end may be parallel with the fixed end.

As used herein, the term "connected" is intended to mean directly connected and indirectly connected. By directly connected, it is intended that the connected elements are in contact with one another or affixed to one another. By indirectly connected, it is intended that one or more intervening or intermediate elements are between the two elements which are secured or "connected" together. The intervening elements may be affixed.

As used herein, the term "line of weakness" is intended to mean any region or area of weakened material, preferably having a length, but not necessarily a defined width. A "line of weakness" can include linear and non-linear patterns, such as curvilinear patterns of weakness, or other shapes, such as circles, rectangles, and so forth. A line of weakness includes a perforation or other series of cuts, a thinning or breakage or separation of material, or a strip of a different kind of material bridging between adjacent portions of material that is more easily torn or broken than the adjacent portions, and which allows the user or manufacturer to separate the adjacent portions along the line of weakness. A line of weakness can further include a single extended slit or cut. Examples of lines of weakness include perforations.

As used herein, the term "polymer" generally includes but is not limited to, homopolymers, copolymers, such as for example, block, graft, random and alternating copolymers, terpolymers, etc. and blends and modifications thereof. Furthermore, unless otherwise specifically limited, the term "polymer" shall include all possible geometrical configuration of the material. These configurations include, but are not limited to isotactic, syndiotactic and random symmetries.

### DETAILED DESCRIPTION OF THE INVENTION

Personal care articles such as, for example, feminine care and incontinent absorbent articles, generally include a liquid pervious topsheet, a substantially liquid impervious backsheet, and an absorbent core positioned and held between the topsheet and the backsheet. The topsheet is generally operatively permeable to the liquids that are intended to be held or stored by the absorbent article, and the backsheet may be substantially impermeable or otherwise operatively impermeable to the liquids intended to be held or stored. Disposable absorbent articles may also include other optional components or layers, such as liquid wicking layers, liquid distribution layers, barrier layers, and the like, as well as combinations thereof, which may improve the fluid handling and storage properties of the disposable absorbent article. Generally, disposable absorbent articles and the components thereof provide a body-facing surface and a garment-facing surface. The body-facing surface is generally the topsheet and garment-facing surface is the backsheet. As an alternative, the substantially liquid impervious backsheet may be replaced with a liquid pervious backsheet, when the absorbent personal care product is used in conjunction with another liquid impervious layer or article, such as, for example liquid impervious pants.

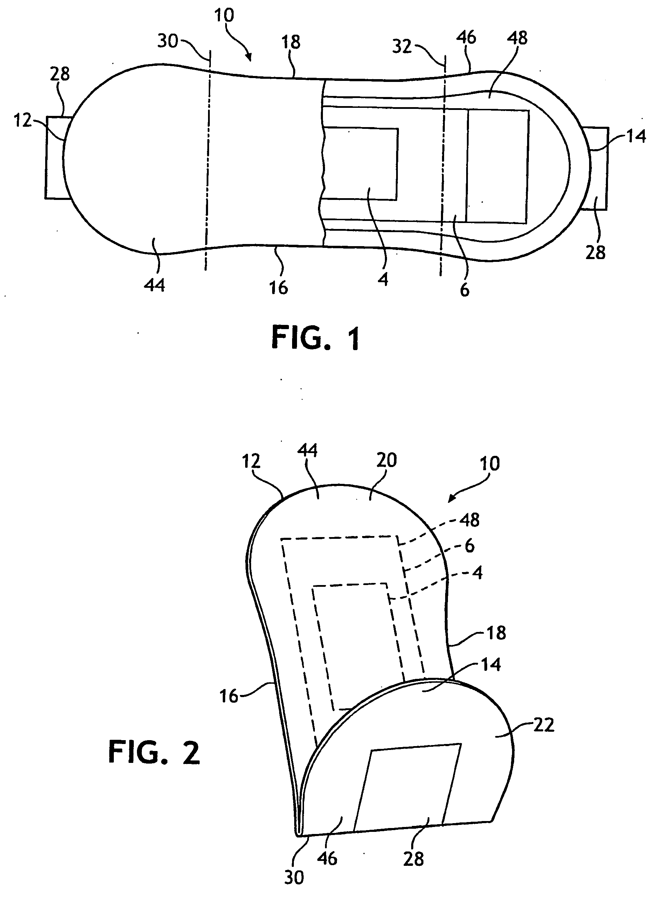

To obtain a better understanding of the absorbent articles which may be present in the packaging component of the present invention, attention is directed to FIGS 1 and 2. In FIGS 1 and 2, an exemplary absorbent product 10 is shown as including an outer cover 46 (otherwise referred to as a baffle or backsheet), an absorbent core 48, an optional tissue layer 6, an optional distribution layer (surge layer) 4 and a body side liner 44 (also referred to as the top sheet). The absorbent product 10 also has a first side 16 and a second side 18. The first and second sides 16, 18, respectively, are the longitudinal sides of the elongated absorbent product. The sides can be contoured, for example in a concave shape, or they can be linear. The sides can further include flaps (not shown) that extend laterally outward. Flaps are known in the art and are shown in, for example, U.S. Patent 6,387,084 issued to VanGompel et al. In one embodiment (not shown), one or more elastic elements are disposed along the sides to form a gasket with the body of the user. Elastic sides are known in the art, as is shown in U.S. Patent 6,315,765 issued to Datta et al. In one embodiment, the elastic elements are disposed between the body side liner and the outer cover.

The absorbent product 10 has a first body side surface 20 and a second garment side surface 22. Applied to at least a portion of the second garment side surface 22 is a garment attachment adhesive. In various embodiments, the garment attachment adhesive is configured as a single band of adhesive or as two or more spaced apart strips. Alternatively, the garment attachment adhesive includes a swirl pattern of adhesive which encompasses a major portion of the second garment surface 22 of the absorbent article 10.

A release strip 28, also known as a releasable peel strip, is removably secured to the garment attachment adhesive and serves to prevent premature contamination of the adhesive before the absorbent article 10 is secured to the crotch portion of an undergarment. In various embodiments, the garment attachment adhesive is designed to be secured to the inner crotch portion of an undergarment so as to keep the absorbent product in register with the body of the user. The release strip 28 may extend beyond one or both of the ends 12, 14 of the outer cover, as shown in FIG 1. Alternatively, the release strip may be as short as the length of the garment attachment adhesive, or slightly longer than the adhesive or may be only as long as the garment attachment adhesive, but does not extend beyond the ends 12 and 14 of the outer cover, as shown in FIG 2.

The body side liner or topsheet 44, which is preferably liquid permeable, may be formed from one or more materials. The body side liner or topsheet 44 must be able to manage different body excretions depending on the type of product. In feminine care products, often the body side liner or body contacting layer must be able to handle menses and urine. In the present invention, the body side liner or topsheet 44 may include a layer constructed of any operative material, and may be a composite material. For example, the body side liner or body contacting layer can include a woven fabric, a nonwoven fabric, a polymer film, a film-nonwoven fabric laminate or the like, as well as combinations thereof. Examples of a nonwoven fabric useable in the body side liner or topsheet 44 include, for example, an airlaid nonwoven web, spunbond nonwoven web, meltblown nonwoven web, a bonded carded web, hydroentangled nonwoven webs, spunlace webs or the like, as well as combinations thereof. Other examples of suitable materials for constructing the body side liner or topsheet 44 can include rayon, bonded carded webs of polyester, polypropylene, polyethylene, nylon, or other heat-bondable fibers finely perforated film webs, net-like materials, and the like, as well as combinations thereof. These webs can be prepared from polymeric materials such as, for example, polyolefins, such as polypropylene and polyethylene and copolymers thereof, polyesters in general, including aliphatic esters such as polylactic acid, nylon or any other heat bondable materials.

Other examples of suitable materials for the body side liner or topsheet 44 are composite materials of a polymer and a nonwoven fabric material. The composite materials are typically in the form of integral sheets generally formed by the extrusion of a polymer onto a nonwoven web, such as a spunbond material. In a desired arrangement, the body side liner or body contacting layer 44 can be configured to be operatively liquid-permeable with regard to the liquids that the article is intended to absorb or otherwise handle. The operative liquid-permeability may, for example, be provided by a plurality of pores, perforations, apertures or other openings, as well as combinations thereof, which are present or formed in the body side liner or body contacting layer. The apertures or other openings can help increase the rate at which bodily liquids can move through the thickness of the body side liner or body contacting layer and penetrate into the other components of the article (e.g. into the absorbent core 48). The selected arrangement of liquid-permeability is desirably present at least on an operative portion of the body side liner or topsheet 44, that is appointed for placement on the body-side of the article. The body side liner or topsheet 44 can provide comfort and conformability, and can function to direct bodily exudates away from the body and toward the absorbent core 48. The body side liner or topsheet 44 can be configured to retain little or no liquid in its structure, and can be configured to provide a relatively comfortable and non-irritating surface next to the body-tissues of a wearer.

The baffle or backsheet 46 may include a layer constructed of any operative material, and may or may not have a selected level of liquid-permeability or liquid-impermeability, as desired. In a particular configuration, the baffle or backsheet 46 may be configured to provide an operatively liquid-impermeable baffle structure. The baffle or backsheet 46 may, for example, include a polymeric film, a woven fabric, a nonwoven fabric or the like, as well as combinations or composites thereof. For example, the baffle may include a polymer film laminated to a woven or nonwoven fabric. In a particular feature, the polymer film can be composed of polyethylene, polypropylene, polyester or the like, as well as combinations thereof. Additionally, the polymer film may be micro-embossed, have a printed design, have a printed message to the consumer, and/or may be at least partially colored. Suitably, the baffle or backsheet 46 can operatively permit a sufficient passage of air and moisture vapor out of the article, particularly out of an absorbent (e.g. storage or absorbent core 48) while blocking the passage of bodily liquids. An example of a suitable baffle material can include a breathable, microporous film, such as those described in, for example, U.S. Patent 6,045,900 to Haffner et al.

Bicomponent films or other multi-component films can also be used, as well as woven and/or nonwoven fabrics which have been treated to render them operatively liquid-impermeable. Another suitable baffle material can include a closed cell polyolefin foam. For example, a closed cell polyethylene foam may be employed.

The liquid permeable body side liner 44 and the liquid-impermeable baffle 46 may be peripherally sealed together to enclose the absorbent core 48 to form the absorbent article 10. Alternatively, the body side liner or topsheet 44 can be wrapped around both the absorbent 48 and the baffle or backsheet 46 to form a wrapped pad. The body side liner 44 and baffle 46, and other components of the absorbent product, can be joined for example with adhesive bonds, sonic bonds, thermal bonds, pinning, stitching or any other attachment technique known in the art, as well as combinations thereof.

The absorbent core 48 is designed to absorb body exudates, including menstrual fluid, blood, urine, and other body fluids. The absorbent core 48 may contain one or more layers of absorbent material. The layers can contain similar materials or different materials. Suitable materials for the absorbent core 48 include, for example, cellulose, wood pulp fluff, rayon, cotton, and meltblown polymers such as polyester, polypropylene or coform. Coform is a meltblown air-formed combination of meltblown polymers, such as polypropylene, and absorbent staple fibers, such as cellulose. A preferred material is wood pulp fluff, for it is low in cost, relatively easy to form, and has good absorbency.

The absorbent core 48 can also be formed from a composite comprised of a hydrophilic material which may be formed from various natural or synthetic fibers, wood pulp fibers, regenerated cellulose or cotton fibers, or a blend of pulp and other fibers. A desired material is an airlaid material.

In one embodiment, the absorbent core 48 also includes a superabsorbent material, in addition to or in place of the hydrophilic material, which increases the ability of the absorbent core to absorb a large amount of fluid in relation to its own weight. Generally stated, the superabsorbent material can be a water-swellable, generally water-insoluble, hydrogel-forming polymeric absorbent material, which is capable of absorbing at least about 15, suitably about 30, and possibly about 60 times or more its weight in physiological saline (e.g. saline with 0.9 wt% NaCl). The superabsorbent materials can be inserted as particles or in sheet form. The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like. Other suitable hydrogel-forming polymers include hydrolyzed acrylonitrile grafted starch, acrylic acid grafted starch, and isobutylene maleic anhydride copolymers and mixtures thereof. The hydrogel-forming polymers may be lightly crosslinked to render the material substantially water insoluble. Crosslinking may, for example, be by irradiation or covalent, ionic, Van der Waals, or hydrogen bonding. Hydroxyfunctional polymers have been found to be good superabsorbents for sanitary napkins. Such superabsorbents are commercially available from Dow Chemical, Hoechst-Celanese, and Stockhausen, Incorporated, among others, and are a partially neutralized salt of cross-linked copolymer of polyacrylic acid and polyvinyl alcohol having an absorbency under load value above 25 grams of absorbed liquid per gram of absorbent material (g/g). Other types of superabsorbent materials known to those skilled in the art can also be used.

Additional layers or substrates, including for example, the liquid acquisition and distribution layer 4, also referred to as a surge or transfer layer, and an optional tissue layer 6 are also incorporated into the absorbent product, for example between the body side liner or topsheet 44 and the absorbent core 48. The distribution layer 4 may be shorter than the absorbent core 48 or have the same length as the absorbent core 48. The distribution layer serves to temporarily hold an insulting fluid to allow the absorbent core sufficient time to absorb the fluid, especially when a superabsorbent material is present. In one embodiment, the absorbent core, transfer layer and other components, such as tissue layers, are free floating (unattached) between the outer cover and the body side liner, which are secured along only the peripheral edges thereof. Alternatively, the absorbent core, transfer layer and other components are attached to one or both of the outer cover and body side liner and/or to each other.

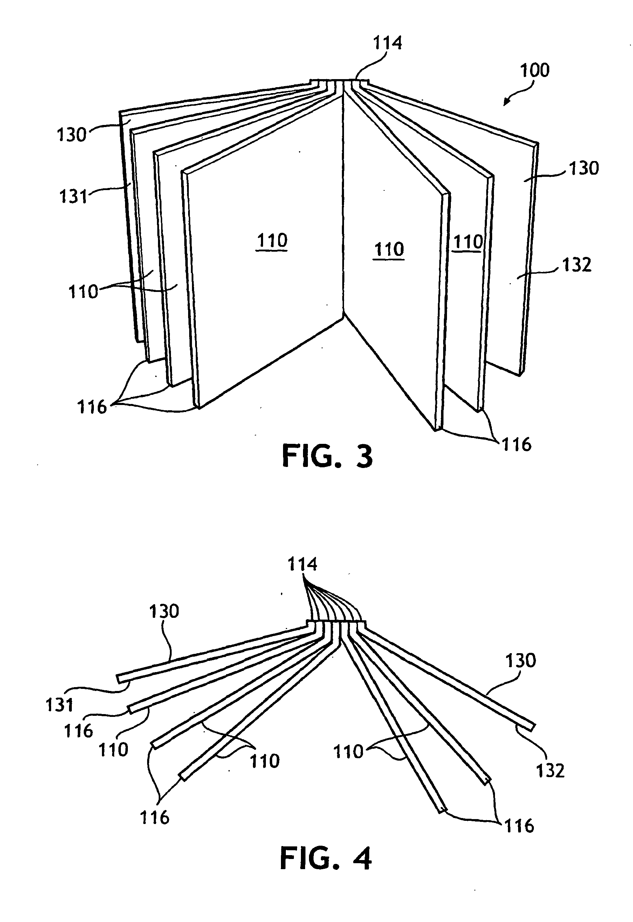

In one embodiment of the present invention, the absorbent article 10 may be in a folded configuration. For example, the absorbent article can be folded along a pair of fold lines 30, 32 to form a tri-fold configuration. In other embodiments, the absorbent article may be bi-folded, flat or rolled. The absorbent product is then inserted into an individual packaging component 110, shown in FIGS 3-6 and 8, as discussed in more detail below.

A package for holding and dispensing absorbent personal care articles, such as those described above, is provided by the present invention. To gain a better understanding for the present invention, attention is directed to FIGS 3 and 4. The package 100 has a plurality of packaging components 110, each packaging component comprising interior portion (not shown), a fixed end 114 and a free end 116. Each of the fixed ends 114 is affixed directly or indirectly to at least one other fixed end 114 of another packaging component 110. Each of the free ends 116 of a given packaging component is opposed to the fixed end 114. In the present invention, each packaging component 110, having at least one absorbent personal care article 10 placed within each interior portion. The package of the present invention has a book-like structure, with the pages of the book being the packaging component 110 with the absorbent article 10 contained therein. The book-like structure allows for easy access of the individual packaging components 110. Also shown in FIGS 3 and 4, is an optional cover 130 which can be placed in front of the first packaging component and behind that last packaging component. The optional cover may be a unitary piece or may be two separate pieces. As shown FIGS 3 and 4, the cover has a first cover 131 (front cover) and a second cover 132 (back cover), where the first cover 131 is in front of the first packaging component and the second cover 132 is behind the last packaging component, in a left to right configuration. It is pointed out that the terms "front cover" and "back cover" are relative terms and are not intended to signify that one side is always the front or top cover and one side is always the back or bottom cover. Stated another way, the back cover could actually be the front of the package and the front cover could be back of the package.

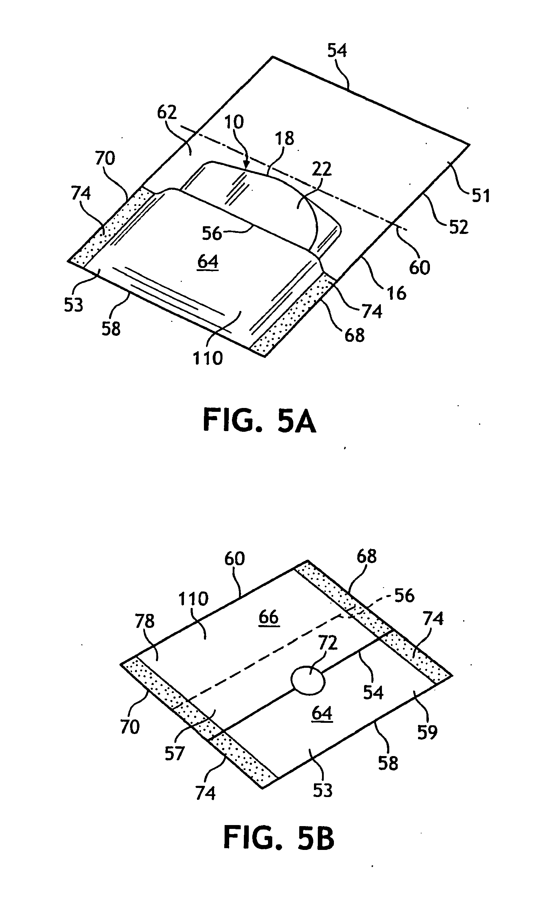

Although the packaging component 110 may have many different configurations or can be prepared in other ways without departing from the scope of the present invention, referring to FIGS 5A and 5B, one typical packaging component 110 has a pouch-like configuration formed from a strip or web 52 of material having a first and second ends having free edge 56, 54, respectively. It should be understood that the term "free edge" refers to an edge that is unattached after the packaging component is opened, regardless of whether the free edge is attached when the packaging component is closed. Each of the first and second ends is folded along fold lines to define the top 60, which is also the free end 116 of the packaging component and bottom edge 58, which is also the fixed end 114 of the packaging component. The folded packaging component has a back panel 62, a first panel 64 and a second panel 66. The first panel 64 and back panel 62 may be secured along side edges 68, 70 thereof to form a pocket shape to receive the absorbent product. Each packaging component has an interior surface 51 and an exterior surface 53. In one embodiment, the pocket and pouch may be shaped and dimensioned to receive a single product component, which is individually wrapped in the pouch. Alternatively, the pocket and pouch may be configured to hold more that one absorbent article. The second panel 66 is folded over the first panel 64 such that the free edge 54 of the second panel overlies the first panel 64. The first panel 64 may have a covered or overlapped portion 57 extending between the free edge 54 (exterior) and the free edge 56 (interior), which covered or overlapped portion 57 underlies the second panel 66. Generally, the second panel may be a flap, but it is not required for the present invention that the second panel be a flap. A flap is a piece of the packaging component which may be manipulated by a user by grasping the end of the second panel and pulling the second panel upward, towards the top 60.

When the second panel overlaps the first panel, in one example, the portion 57 has a length of about 0 about 50 mm, generally about 2-22 mm and typically about 4-10 mm between the free edges 54, 56. In various embodiments, the overlap distance is less than or equal to about 95% of the overall packaging component length in a closed configuration, more desirably less than or equal to about 35% of the packaging component length, and more desirably less than or equal to about 20% of the packaging component length. In various embodiments, the free edge 54 is positioned a distance from either edge 58, 60 that is greater than or equal to about 10% of the overall length of the packaging component (in a closed configuration), more desirably greater than or equal to about 30%, and more desirably about 50% of the packaging component length. The first panel further includes an uncovered second portion 59 extending between the free edge 54 and the bottom edge 58. Of course, it should be understood that the length and width of the article and packaging components can vary according to the type of article and the size of the article.

A pair of side seals 74 secures the first panel 64 to the back panel 62. The side seals are desirably formed after the first panel is folded over the back panel and the second panel is folded over the back panel and the first panel. Although, it is possible that the first panel could first be sealed to the back panel, and the second panel then sealed to one or both of the back panel and first panel. The sides may be sealed by any method known to those skilled in the art. Exemplary sealing methods include, for example, adhesive sealing, bonding by the application of heat and pressure, ultrasonic bonding or any other art-known bonding methods. In one embodiment of the present invention, the side seals 74 may be frangible, meaning they can be easily broken such that the second panel 66 can be separated from the first panel 64 and back panel 62, and such that the first panel 64 can be easily separated from the back panel 62, wherein the product component 10 is exposed for removal from the pouch by the user.

In an alternative configuration, one or more free edges may be formed along a perforation line, which is or may be adhered to an underlying layer, with the edge defined by the perforation line being a "free edge" after the perforation line is broken.

The packaging component material can be formed from materials, such as, but not limited to, a non-woven material, films, paper, laminates, and/or cloth (including woven) materials, and combinations thereof. For example, the pouch can be made as disclosed in U.S. Patent 6,716,203, to Sorebo et al. Suitable laminates useable in the present invention include, spunbond-spunbond laminate (SS), spunbond-meltblown-spunbond laminates (SMS), spunbond-film laminates (SF), and film-film laminates. In one embodiment, the pouch is made of a film/spunbond laminate material available from Kimberly-Clark Corp, and known as HBSTL ("highly breathable stretch thermal laminate"), and which material is further disclosed in U.S. Patent No. 6,276,032, to Nortman et al.

In one alternative embodiment, the second panel 66 is releasably secured to the first panel 64.. For example, a fastening element 72, shown as a tab in FIG 5B, is secured across the free edge 54 of the second panel 66 to secure the second panel 66 to the first panel 64. The fastening element can be releasably secured to both of the second panel and first panel, or it can be fixedly secured to one of the second panel and first panel and releasably secured to the other. Other possible configurations include that the fastening element is fixedly secured to both panels and one or both of the panels is provided with an area of weakness, such as a perforated area, which allows a portion of one or both of the panels to be removed or damaged when the packaging component is opened. The fastening element can be formed as adhesive tape, a snap, a button, a mechanical fastener (e.g., hook and loop), a tie, or as any other device known by those skilled in the art. The fastening element can have various alternative shapes, including but not limited to a square, rectangle, triangle, circle, oval, obround, oblong or diamond shape, or any other irregular shape or pattern. In an alternative embodiment, the fastening element is formed on the inside of the second panel such that it engages the first panel as the second panel is folded thereover and is not visible to the user. For example, the fastening of the second panel 66 to the first panel 64 may be accomplished by the use of an adhesive applied to the side of the second panel 66 which contacts the first panel 64, to the side of the first panel 64 which contacts the second panel 66, or both. This adhesive may be applied as a ribbon, dot, a swirl pattern or any other pattern which effectively adheres the second panel 66 to the first panel 64. In another alternative way to fasten the second panel 66 to the first panel 64, the second panel 66 is simply sealed to the first panel 64 with a heat seal or other weld, with the weld defining the fastening element In another embodiment, the second panel 66 is not sealed or otherwise attached to the first panel 64, but rather is simply folded thereover. Alternatively, the sides of the second panel are sealed to the back panel and to the first panel, with the side seals being breakable in response to a user grasping and lifting the second panel.

Each of the individual packaging components 110 is designed in such a way that each packaging component 110, is removable from the package 100. Each of these configurations will be discussed in more detail below. Either of the side edges 68, 70, or either the top edge 60 or the bottom edge 58 may be located proximate to the fixed end 114 of the packaging component 110 with the opposite edge being the free end 116 of the packaging component 110. However, from an ease of access to the absorbent article 10 within the package component 110, it is better if one of the side edges 68, 70, or the bottom edge 58 is proximate to the fixed end, since it could be difficult to remove the article 10 from the top of the packaging component 110 if the top 60 is proximate to the fixed end 114.

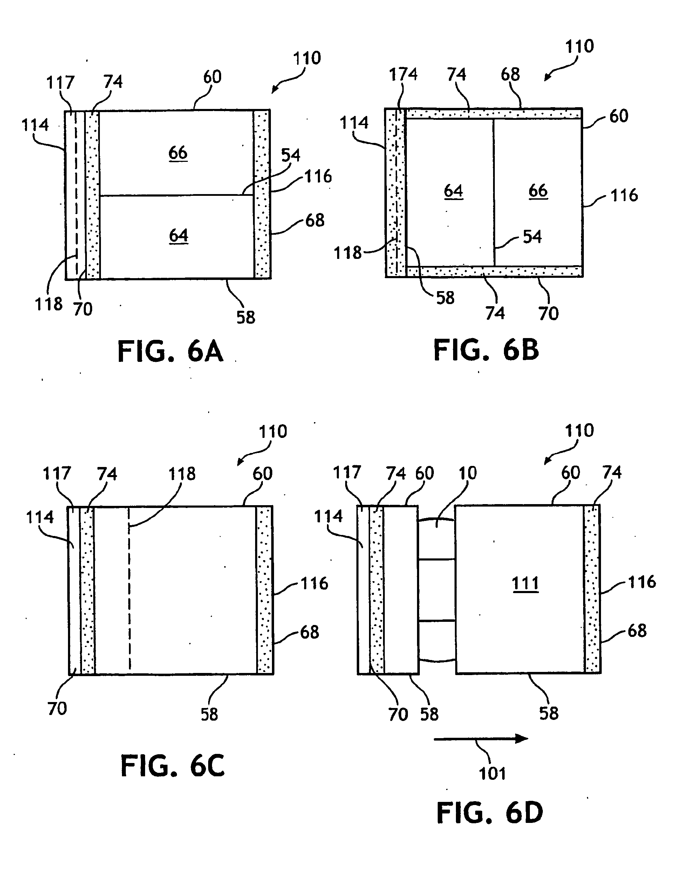

Referring to FIG 6A, the packaging component 110 has an affixed end 114, which is proximate to the edge 70. In this configuration, the other edge 68 is located at the free end 116 of the packaging component. Also shown in FIG 6A, the packaging component may have an optional tab 117 of material which is made from the packaging material. The optional tab 117 may serve as an attachment location or attachment area of each individual packaging component to other packaging components 110 within the package 100. From an ease of opening the packaging component 110 standpoint, however, it is desired that the tab of material is present on the packaging component. However, the tab 117 is not necessary and the packaging component 110 may be affixed to the other packaging components in the seal area 74. Another option is to provide a seal area 74 which is wider near the edge which is proximate to the fixed end. This would provide a larger location to affix each packaging component 110 to other packaging components.

Also shown in FIG 6A is an optional line of weakness 118 present on the tab 117. The line of weakness 118, when present, allows the packaging component 110, with the article 10 contained therein, to be removed or dislodged from the package 100, while retaining the other packaging components 110 attached to the package 100. For example, the free end could be subjected to a tension force which would cause the line of weakness to break and allow a portion of the packaging component to be removed. Generally, the line of weakness 118 is in the form of perforations or other such structure which will allow the user to tear the line of weakness without damaging the packaging component 110, causing the article 10 to be removed from the packaging component 110, damaging the package 100 or causing the other packaging components 110 to become dislodged from the package. As an alternative to having the line of weakness on the tab, the line of weakness could be located within the seal area 74 proximate to the fixed end 114, shown in FIG 6B. Other methods of rendering the packaging components 110 removable from the package can also be used, besides using a line of weakness, which will be discussed in more detail below.

An alternative configuration for the packaging component 110 is shown in FIG 6B. In this figure, the bottom edge 58 is proximate to the fixed end 114. It is pointed out that the top edge 60 is located at the free end 116 of the packaging component 110. As a result of this configuration, the article is removed from the packaging component towards the free end 116 of the packaging component 110. In the configuration shown in FIG 6B, similar to the configuration shown in FIG 6A, the packaging component may have an optional tab 117 of material (not shown in FIG 6B) which is made from the packaging component material. As an optional feature, the bottom 58 of the packaging component 110 may also have a seal area 174, which is similar to the seal area 74. By having the optional seal area 174, the packaging component 110 may be removed from the package 110 without the package being compromised in such a way as to result in the absorbent article becoming soiled or contaminated from an opening being formed in the packaging component 110, when the packaging component 110 is removed from the package 100. As is shown in FIG 6B, a line of weakness 118 may be provided in the seal area 174, which allows the packaging component 110 to be removed from the package 100. It is not necessary for the line of weakness 118 to be located in the seal area 174, and the configuration of FIG 6B may also have an optional tab 117 area (not shown in FIG 6B), as is shown in FIG 6A. As with the configuration of FIG 6A, other methods of rendering the packaging component removable or detachable form the package may be used.

In another alternative configuration outside the scope of the claims, the packaging component 110 could become opened as the packaging component 110 is pulled or otherwise removed from the package 100, thereby dispensing the article at the same time. In this regard, attention is directed to FIG 6C and 6D. As shown in these figures, a portion 111 of the individual packaging component 110 may be removable. When the free end 116 of the packaging component is pulled in order to remove the packaging component 110 and article 10 from the package, a portion 111 of the packaging component is caused to be removed. As a result, the article 10 is caused to be dispensed from the packaging component 110 and the package 100. In one embodiment of this configuration, the packaging component has a line of weakness 118 which is located between the fixed end 114 and the free end 116 and generally on the packaging component in a location proximate to the article 10 within the packaging component 110. Generally, the line of weakness 118 is away from the fixed end 114 and may be as close to the free end 116 such that the line of weakness is just inside the free end 116. As the line of weakness is torn or compromised, such as by applying a force 101 in a direction away from the fixed end 114, a portion 111 of the packaging component 110 becomes free at the line of weakness. As a result, as the portion 111 of the packaging component becomes free, the article 10 becomes accessible from the packaging component 110 and package 100. This is shown in FIG 6D.

The individual packaging components 110 may be attached directly to each other at the fixed end 114, such that the fixed end has an attachment area. The attachment area is generally on the optional tab 117 or on the seal area 74 or 174, as shown in the FIGS 6A-6B. In the alternative, each packaging component 110 may be indirectly connected to the other packaging components 110.

The packaging components may be directly attached to one another, as is shown in FIG 4. To obtain this configuration, the fixed end 114 in the seal area 74 or the tab 117 are brought into contact with each other and bonded together. Any bonding method which will effectively hold the packaging components together may be used, without departing from the scope of the present invention. Suitable bonding methods include, for example, adhesive sealing, bonding by the application of heat and pressure, ultrasonic bonding or any other art-known bonding methods. Examples of adhesives include hot melt adhesives, solvent based adhesive and the like.

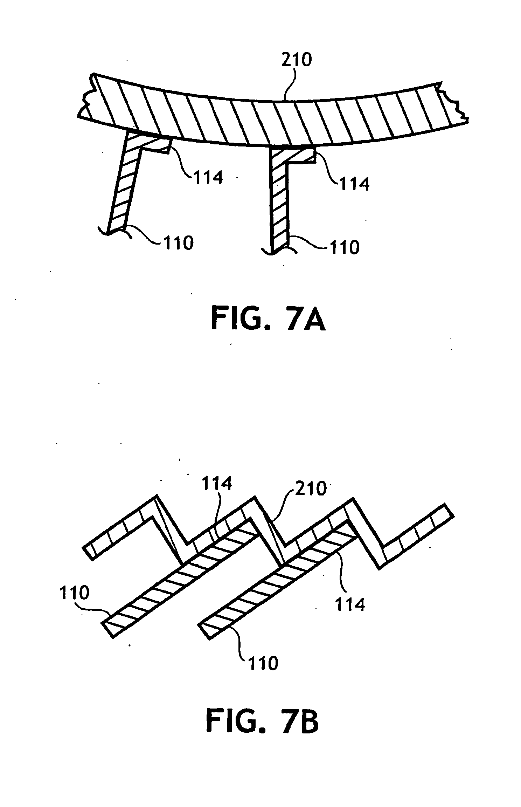

In the alternative, the packaging components 110 may be indirectly attached to other packaging components 110. For example, as is shown in FIG 7A and 7B, the fixed end 114 of the packaging component 110 is connected or otherwise attached to an additional material 210. One method, which is shown in FIG 7A would be to fold a portion of the tab or seal area to increase the area of attachment. This is shown in FIG 7A. This additional material may be any material which will allow the packaging components to be attached thereto. In one embodiment, the additional material is one which allows for the package to compress or become smaller when, as the absorbent articles 10 or packaging components 110 are removed from the package. As such, the material in which the packaging components are attached may be an elastic material, a corrugated material, which is shown in FIG 7B or any other type of material that will contract as the packaging components 110 and/or the absorbent articles are removed. In another embodiment, the material which the packaging components are attached may be a rigid or pliable material, which does not contract. Suitable materials usable for holding the packaging components together include, nonwoven webs, paper, paperboard, woven or knitted webs and the like.

In addition, other connection means may be used. For example, mechanical connecting means such as ring binders, spiral binders, pins or the like may also be used to connect the packaging component pages 110 together. With the foregoing connecting means, apertures may be punched in the tab 117 or the seal areas 74, 174 in order for the mechanical attachment to have a way to hold the packaging component pages together. With these mechanical attachment means, the apertures may also provide a means for removing an individual packaging component 110 from the package, much in the same way as a piece of paper is removed from a notebook. These mechanical attachment means may provide an advantage over other attachment means in that as the articles are used, the size of the overall package is reduced, since the entire page of the wrapper component may be removed. Other mechanical methods such as clips or similar means to hold the packaging components together may also be used.

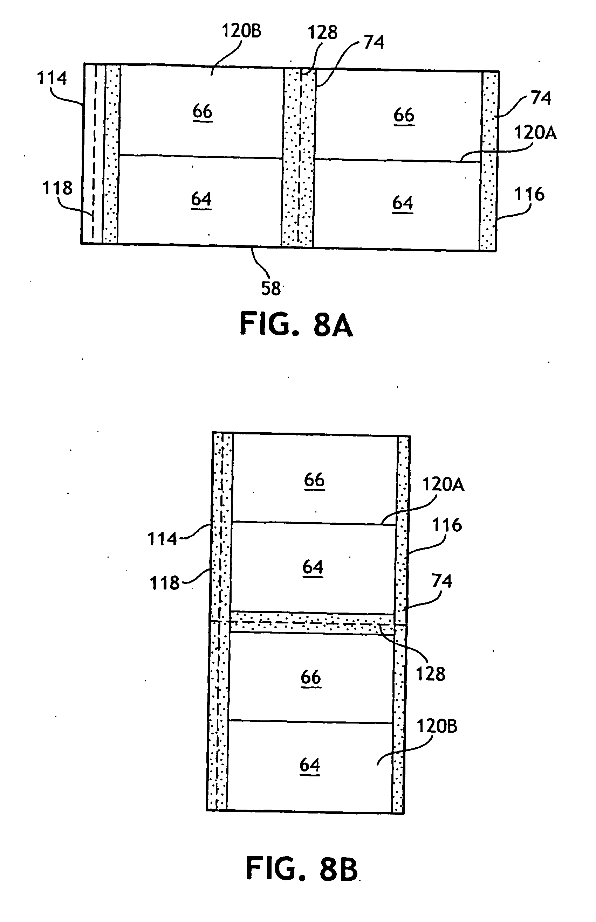

In the FIGS 3-6, the figures show that the packaging component has a single compartment on a given "page" of the book-like package 100. However, there could be more than one compartment on a given page or packaging component 110 of the package 100. Attention is directed to FIGS 8A and 8B, which both show possible configurations for a packaging component 110 having more than one compartment capable of holding absorbent articles. In FIG 8A, the two compartments 120A and 120B are side-by-side on the packaging component, meaning that in a line perpendicular to the fixed end 114 to the free end 116,will cross two compartments. In FIG 8B which is outside the scope of the claims, the two compartments 120A and 120B are arranged one on top of the other, meaning that a line parallel to the free end 116 and the fixed end 114 will cross the two compartments. Each of the compartments may be separated by a means which will allow the compartments to be separated without opening or otherwise compromising the compartment and the absorbent personal care article contained therein. For example, and as is shown in FIG 8A and FIG 8B, a line of weakness 128 or other means may be used to separate the individual wrappers from a given page of the packaging component. Generally, the packaging component 110 with more than one compartment may have the features of the single compartment packaging component described above. Each of the compartments of the packaging component may be configured such that there is more than one absorbent article accessible in the compartments or be configured to hold a single absorbent article. In addition, the packaging component may also be configured such that there are more than two compartments in a given packaging component page 110 of the package 100.

Each package may have any number of packaging components 110 and absorbent articles 10. Generally, there are between about 2 and about 100 packaging component pages and/or absorbent articles in a given package. Often, there are between 3 and 50 packaging component pages in the package. From a standpoint of bulkiness, it is generally desired that there are less that 20 packaging component 110 pages in the package 100. The overall thickness (bulkiness) of the package and the number of absorbent articles within a given package is dependent on factors including, for example, the thickness of the absorbent articles contained within the packaging component. With thinner products, more absorbent articles can be stored in the package, without the package being too thick. Generally, the overall thickness should be less than the thickness in which an average user of the products can grasp with one hand.

Referring back to FIGS 3 and 4, the package may also have a cover 130 which helps protect the packaging components from damage during storage, transport and on store shelves. The cover may be a unitary material or may be multiple pieces of material. In one configuration shown in FIGS 3 and 4, the cover 130 is composed of a first cover 131 and a second cover 132. As shown in these figures, the cover is more that one piece. The cover may also be a unitary material in which the packaging component pages 110 are attached directly to the cover material in a region between the front portion of the cover and the back portion of the cover. The cover material may be prepared from a nonwoven web, paper, paperboard, cardboard, a woven or knitted web, a polymeric material, and the like. The cover could also serve as a place to insert product information, for example, instructions of how to remove the packaging components or absorbent articles from the packaging components.

With or without the cover, the package may also be provided with a closing means or fastening element to keep the package in a closed position. The closing means or fastening element can be releasably secured to both of the first cover and the second cover, or it can be fixedly secured to one of the first and second covers and releasably secured to the other. If the cover is not present, the fastening system may be applied to the first and last packaging components in the package. The fastening element can be formed as an adhesive tape, a snap, a button, a mechanical fastener (e.g., hook and loop), a tie, a spring loaded fastening element, a magnetic fastening element, an elastic band fastening element, or any other device known by those skilled in the art which can fasten the front cover to the back cover or the first page to the last page. In addition, the fastening element may also perform the function of a means for hanging the package on a vertical surface or non-horizontal, such as a wall, a cabinet door or a passage door, which is discussed below, or a means for securing the package to a horizontal surface, such as a drawer or cabinet shelf, which is also discussed below.

The package of the present invention may be hung on a vertical or other non-horizontal surface such as a wall, a cabinet door or a passage door and the like, or may be laid in a fairly flat configuration, much like an open book. In this configuration, the package may be placed in a cabinet or a drawer for easy access to the absorbent articles and packaging component, when needed. The hanging means or securing means can be any means know to those skilled in the art including, for example, adhesives, mechanical fasteners (e.g.,'hook and loop), magnetic fasteners and the like. As a result, the package provides a way to store wrapped absorbent articles in a confined location without the associated problems of storing loose absorbent articles, in or out of a wrapper type component, such as the articles and wrapper component becoming scattered in a drawer, purse, or other similar location.

## Claims

1. A package (100) for holding and dispensing absorbent personal care articles (10) comprising:
a plurality of absorbent personal care articles (10); and
a plurality of packaging components (110), each packaging component (110) comprising a compartment, a fixed end (114) and a free end (116), wherein said compartment is a pouch formed from a strip of material (52) folded to define a top edge (60), a bottom edge (58), a back panel (62), a first panel (64), a second panel (66) and side edges (68, 70), said second panel (66) being folded over said first panel (64) such that a free edge (54) of said second panel (66) overlies the first panel (64), said first and second panels (64, 66) being secured to said back panel (62) along side edges (68, 70) to form side seals (74), wherein each fixed end (114) is connected directly or indirectly to at least one other fixed end (114) of another packaging component (110) and each free end (116) of a given packaging component (110) is opposed to the fixed end (114), each packaging component (110) having at least one absorbent personal care article (10) place within each compartment, whereby said package (100) forms a book-like structure, wherein:
said component (110) comprises a line of weakness (118) proximate to the fixed end (114) that allows the packaging component (110), with the at least one personal care article (10) contained therein, to be removed from the package (100) while retaining the other packaging components (110) attached to the package (100), wherein said line of weakness (118) is formed on:
one of said side seals (74);
on an additional seal area (174) along said bottom edge (58); or
on a tab of material (117) extending from one of said side seals (74) or from an additional seal area (174) along said bottom edge (58); and
there are between 3 and 50 packaging components in the package.

2. The package (100) of claim 1, further comprising a cover (130), wherein the cover (130) comprises a fixed end (114) and a free end (116), and the cover (130) is directly or indirectly connected to the fixed end (114) of the packaging components (110).

3. The package (100) of claim 2, wherein the cover (130) comprises a front cover (131) and a back cover (132), the front cover (131) and the back cover (132) each have a fixed end (114) and a free end (116), the fixed end (114) of the front cover (131.) is directly or indirectly attached to the fixed end (114) of the back cover (132), and the plurality of packaging components (110) are positioned between the front and back covers (131,132).

4. The package (100) of claim 3, wherein the cover (130) further comprises a closing means (72).

5. The package (100) of claim 4, wherein the closing means comprises an adhesive tape, a snap, a button, a hook and loop fastener a tie, a spring loaded fastening element, a magnetic fastening element, and/ or an elastic band fastening element,

6. The package (100) of any preceding claim, wherein each packaging component (110) is prepared from a material comprising nonwoven fabric, a film, paper, a woven or knitted fabric, a laminate of one or more of the preceding materials, or combinations thereof.

7. The package (100) of any of claims 1 to 5, wherein the packaging component (110) comprises a material selected from the group consisting of a non-woven web, a film, paper, a woven or knitted web, laminates thereof and combinations thereof.

8. The package (100) of any preceding claim, wherein each packaging component (110) comprises at least two compartments (120A,120B), each compartment (120A,120B) comprising at least one absorbent personal care article (10).

9. The package (100) of claim 8, wherein the compartments (120A, 120B) of the packaging component (110) are separated by a means (128) which allows the compartments (120A, 120B) to be separated without opening or otherwise compromising the compartment (120A,120B) and the absorbent personal care article (10) contained therein.

10. The package (100) of any preceding claim, wherein the packaging component (110) is removable from the package (100) by subjecting the free end (114) of the packaging component (110) to a tension force.

11. The package (100) of any preceding claim, wherein the individual packaging components (110) are directly connected to at least one other packaging component (110).

12. The package (100) of any of claims 1 to 10, wherein the packaging components (110) are indirectly connected to the other packaging components (110).

13. The package (100) of claim 12, wherein the packaging components (110) are indirectly connected by an additional material (210), each individual packaging component (110) being attached directly to the additional material (210).

14. The package (100) of any preceding claim, wherein the package (100) further comprises a means for attaching the package (100) to a vertical or non-horizontal surface.

15. The package (100) of any of claims 1 to 13, wherein the package (100) further comprises a means for securing the package (100) to a horizontal surface.

## Patentansprüche

1. Verpackung (100) zum Halten und Abgeben von absorbierenden Körperpflegeartikein (10), welche umfasst:
eine Vielzahl von absorbierenden Körperpflegeartikeln (10); und
eine Vielzahl von Verpackungskomponenten (110); wobei jede Verpackungskomponente (110) ein Fach, eine festes Ende (114) und ein freies Ende (116) umfasst, wobei das Fach ein Tasche ist, die aus einem Streifen von Material (52) gebildet ist, welcher gefaltet ist, um einen oberen Rand (60), einen unteren Rand (58), eine Rückbahn (62), eine erste Bahn (64), eine zweite Bahn (66) und Seitenränder (68, 70) zu bestimmen, wobei die zweite Bahn (66) über die erste Bahn (64) gefaltet ist, so dass ein freies Ende (54) der zweiten Bahn (66) mit der ersten Bahn (64) überlagert, wobei die erste und zweite Bahn (64, 66) an der Rückbahn (62) entlang von Seitenrändern (68, 70) befestigt sind, um Seitendichtungen (74) zu bilden, wobei jedes feste Ende (114) direkt oder indirekt mit mindestens einem anderen festen Ende (114) einer anderen Verpackungskomponente (110) verbunden ist, und wobei jedes freie Ende (116) einer bestimmten Verpackungskomponente (110) dem festen Ende (114) gegenüberliegt, wobei jede Verpackungskomponente (110) mindestens einen absorbierenden Körperpflegeartikel (10) aufweist, welcher jeweils in einem Fach angeordnet ist, wobei die Verpackung (100) eine Buchähnliche Struktur bildet,
wobei die Komponente (110) eine Schwächungslinie (118) in der Nähe des festen Endes (114) umfasst, die es ermöglicht, dass die Verpackungskomponente (110) mit dem darin enthaltenen mindestens einen Körperpflegartikel (10) aus der Verpackung (100) entfernt werden kann, während die anderen Verpackungskomponenten (110) an der Verpackung (100) befestigt bleiben, wobei die Schwächungslinie (118) gebildet ist auf:
einer der Seitendichtungen (74);
auf einem zusätzlichen Dichtungsbereich (174) entlang des unteren Rands (58); oder
auf einer Lasche von Material (117), die sich von einer der Seitendichtungen (74) erstreckt oder von einem zusätzlichen Dichtungsbereich (174) entlang des unteren Rands; und
wobei zwischen 3 und 50 Verpackungskomponenten in der Verpackung vorliegen.

2. Die Verpackung (100) gemäß Anspruch 1, welche des Weiteren einen Deckel (130) umfasst, wobei der Deckel (130) ein festes Ende (114) und ein freies Ende (116) umfasst, und der Deckel (130) direkt oder indirekt mit dem festen Ende (114) der Verpackungskomponenten (110) verbunden ist.

3. Die Verpackung (100) gemäß Anspruch 2, wobei der Deckel (130) einen vorderen Deckel (131) und einen hinteren Deckel (132) umfasst, wobei der vordere Deckel (131) und der hintere Deckel (132) jeder ein festes Ende (114) und ein freies Ende (116) aufweisen, wobei das feste Ende (114) des vorderen Deckels (131) direkt oder indirekt mit dem festen Ende (114) des hinteren Deckels (132) verbunden ist, und die Vielzahl an Verpackungskomponenten (110) zwischen dem vorderen und hinteren Deckel (131, 132) angeordnet sind.

4. Die Verpackung (100) gemäß Anspruch 3, wobei der Deckel (130) des Weiteren ein Verschlussmittel (72) aufweist.

5. Die Verpackung (100) gemäß Anspruch 4, wobei das Verschlussmittel ein Klebetape, einen Druckknopf, einen Knopf, einen Klettverschluss, ein Band, ein Federverschlusselement, ein magnetisches Verschlusselement und/oder einen elastischen Bandverschluss umfasst.

6. Die Verpackung (100) gemäß einem der vorherigen Ansprüche, wobei jede Verpackungskomponente (110) aus einem Material hergestellt ist, welches Vliesstoff, einen Film, Papier, einen gewebten oder gestrickten Stoff, ein Laminat aus einem oder mehreren der vorgenannten Materialen oder Kombination davon umfasst.

7. Die Verpackung (100) gemäß einem der Ansprüche 1 bis 5, wobei die Verpackungskomponente (110) ein Material umfasst, welches ausgewählt ist aus der Gruppe bestehend aus einer Vliesbahn, einem Film, Papier, einer gewebten oder gestrickten Bahn, Laminaten davon und Kombinationen davon.

8. Die Verpackung (100) gemäß einem der vorherigen Ansprüche, wobei jede Verpackungskomponente (110) mindestens zwei Fächer (120A, 120B) umfasst, wobei jedes Fach (120A, 120B) mindestens einen Körperpflegeartikel (10) umfasst.

9. Die Verpackung (100) gemäß Anspruch 8, wobei die Fächer (120A, 120B) der Verpackungskomponente (110) durch Mittel (128) getrennt sind, welche ermöglichen, dass die Fächer (120A, 120B) ohne Öffnen oder sonstiges Beeinträchtigen der Fächer (120A, 120B) und des absorbierenden Körperpflegeartikels (10), der darin enthalten ist, getrennt werden.

10. Die Verpackung (100) gemäß einem der vorherigen Ansprüche, wobei die Verpackungskomponente (110) von der Verpackung (100) durch Aussetzen des freien Endes (114) der Verpackungskomponente (110) einer Zugkraft abnehmbar ist.

11. Die Verpackung (100) gemäß einem der vorherigen Ansprüche, wobei die einzelnen Verpackungskomponenten (110) direkt mit mindestens einer anderen Verpackungskomponente (110) verbunden sind.

12. Die Verpackung (100) gemäß einem der Ansprüche 1 bis 10, wobei die Verpackungskomponenten (110) indirekt mit den anderen Verpackungskomponenten (110) verbunden sind.

13. Die Verpackung (100) gemäß Anspruch 12, wobei die Verpackungskomponenten (110) indirekt durch ein zusätzliches Material (210) verbunden sind, wobei jede einzelne Verpackungskomponente (110) direkt mit dem zusätzlichen Material (210) verbunden ist.

14. Die Verpackung (100) gemäß einem der vorherigen Ansprüche, wobei die Verpackung (100) des Weiteren ein Mittel zum Befestigen der Verpackung (100) an einer vertikalen oder nicht-horizontalen Oberfläche umfasst.

15. Die Verpackung (100) gemäß einem der Ansprüche 1 bis 13, wobei die Verpackung (100) des Weiteren ein Mittel zum Sichern der Verpackung (100) an einer horizontalen Oberfläche umfasst.

## Revendications

1. Paquet (100) pour contenir et distribuer des articles de soins personnels absorbants (10), comprenant:
une pluralité d'articles de soins personnels absorbants (10); et
une pluralité de composants d'emballage (110), chaque composant d'emballage (110) comprenant un compartiment, une extrémité fixe (114) et une extrémité libre (116), ledit compartiment étant une poche formée d'une bande de matériau (52) pliée de manière à définir un bord supérieur (60), un bord inférieur (58), un panneau arrière (62), un premier panneau (64), un deuxième panneau (66) et des bords latéraux (68, 70), ledit deuxième panneau (66) étant plié par-dessus ledit premier panneau (64) de telle sorte qu'un bord libre (54) dudit deuxième panneau (66) recouvre le premier panneau (64), lesdits premier et deuxième panneaux (64, 66) étant fixés audit panneau arrière (62) le long de bords latéraux (68, 70) pour former des joints latéraux (74), chaque extrémité fixe (114) étant connectée directement ou indirectement à au moins une autre extrémité fixe (114) d'un autre composant d'emballage (110), et chaque extrémité libre (116) d'un composant d'emballage donné (110) étant opposée à l'extrémité fixe (114), chaque composant d'emballage (110) contenant au moins un article de soins personnels absorbant qui est placé à l'intérieur de chaque compartiment, avec comme résultat que ledit paquet (100) forme une structure similaire à celle d'un livre,
ledit composant (110) comprenant une ligne d'affaiblissement (118) à proximité de l'extrémité fixe (114), qui permet au composant d'emballage (110), avec l'au moins un article de soins personnels absorbant (10) contenu dans celui-ci, d'être retiré du paquet (100) tout en conservant les autres composants d'emballage (110) attachés au paquet (110), ladite ligne d'affaiblissement (118) étant formée :
sur l'un desdits joints latéraux (74) ;
sur une zone de joint supplémentaire (174) le long dudit bord inférieur (58) ; ou
sur une languette de matériau (117) s'étendant depuis l'un desdits joints latéraux (74) ou depuis une zone de joint supplémentaire (174) le long dudit bord inférieur (58) ; et
de 3 à 50 composants d'emballage étant prévus dans le paquet.

2. Paquet (100) selon la revendication 1, comprenant en outre une couverture (130), dans lequel la couverture (130) comprend une extrémité fixe (114) et une extrémité libre (116), et la couverture (130) est connectée directement ou indirectement à l'extrémité fixe (114) des composants d'emballage (110).

3. Paquet (100) selon la revendication 2, dans lequel la couverture (130) comprend une couverture avant (131) et une couverture arrière (132), la couverture avant (131) et la couverture arrière (132) présentent chacune une extrémité fixe (114) et une extrémité libre (116), l'extrémité fixe (114) de la couverture avant (131) est directement ou indirectement attachée à l'extrémité fixe (114) de la couverture arrière (132), et la pluralité de composants d'emballage (110) sont positionnés entre les couvertures avant et arrière (131, 132).

4. Paquet (100) selon la revendication 3, dans lequel la couverture (130) comprend en outre des moyens de fermeture (72).

5. Paquet (100) selon la revendication 4, dans lequel les moyens de fermeture comprennent un ruban adhésif, une pression, un bouton, une fermeture velcro, un lien, un élément de fixation à ressort, un élément de fixation magnétique et/ou un élément de fixation à bande élastique.

6. Paquet (100) selon l'une quelconque des revendications précédentes, dans lequel chaque composant d'emballage (110) est préparé à partir d'une matière comprenant un tissu non tissé, un film, un papier, un tissu tissé ou tricoté, un stratifié d'une ou de plusieurs des matières précitées, ou des combinaisons de celles-ci.

7. Paquet (100) selon l'une quelconque des revendications 1 à 5, dans lequel le composant d'emballage (110) comprend une matière qui est sélectionnée dans le groupe comprenant une bande non tissée, un film, un papier, une bande tissée ou tricotée, des stratifiés de ces matières et des combinaisons de celles-ci.

8. Paquet (100) selon l'une quelconque des revendications précédentes, dans lequel chaque composant d'emballage (110) comprend au moins deux compartiments (120A, 120B), chaque compartiment (120A, 120B) comprenant au moins un article de soins personnels absorbant (10).

9. Paquet (100) selon la revendication 8, dans lequel les compartiments (120A, 120B) du composant d'emballage (110) sont séparés par un moyen (128) qui permet de séparer les compartiments (120A, 120B) sans ouvrir ou compromettre autrement le compartiment (120A, 120B) et l'article de soins personnels absorbant (10) contenu dans celui-ci.

10. Paquet (100) selon l'une quelconque des revendications précédentes, dans lequel le composant d'emballage (110) peut être sorti du paquet (100) en soumettant l'extrémité libre (114) du composant d'emballage (110) à une force de traction.

11. Paquet (100) selon l'une quelconque des revendications précédentes, dans lequel les composants d'emballage individuels (110) sont directement connectés à au moins un autre composant d'emballage (110).

12. Paquet (100) selon l'une quelconque des revendications 1 à 10, dans lequel les composants d'emballage (110) sont indirectement connectés aux autres composants d'emballage (110).

13. Paquet (100) selon la revendication 12, dans lequel les composants d'emballage (110) sont indirectement connectés par une matière supplémentaire (210), chaque composant d'emballage individuel (110) étant attaché directement à la matière supplémentaire (210).

14. Paquet (100) selon l'une quelconque des revendications précédentes, dans lequel le paquet (100) comprend en outre des moyens pour attacher le paquet (100) à une surface verticale ou non horizontale.

15. Paquet (100) selon l'une quelconque des revendications 1 à 13, dans lequel le paquet (100) comprend en outre des moyens pour fixer le paquet (100) à une surface horizontale.
